# EUROPÄISCHE PATENTSCHRIFT

(11) **EP 4 081 331 B1**
(45) Veröffentlichungstag und Bekanntmachung des Hinweises auf die Patenterteilung: **22.01.2025**
(21) Anmeldenummer: 20835750.9
(22) Anmeldetag: 16.12.2020
(51) Int. Cl.: B01D 65/10, G01N 33/00

(54) **VERFAHREN UND VORRICHTUNG ZUR ANALYSE EINER FUNKTIONALEN SCHICHT EINER ELEKTROCHEMISCHEN ZELLE ODER EINER ELEKTROCHEMISCHEN SENSORENANWENDUNG**
METHOD AND APPARATUS FOR ANALYSING A FUNCTIONAL LAYER OF AN ELECTROCHEMICAL CELL OR AN ELECTROCHEMICAL SENSOR APPLICATION
PROCÉDÉ ET DISPOSITIF D'ANALYSE D'UNE COUCHE FONCTIONNELLE D'UNE CELLULE ÉLECTROCHIMIQUE OU D'UNE APPLICATION DE CAPTEUR ÉLECTROCHIMIQUE

(30) Priorität: 23.12.2019 DE 102019220561
(43) Veröffentlichungstag der Anmeldung: 02.11.2022
(73) Patentinhaber: Greenerity GmbH, 63457 Hanau-Wolfgang (DE)
(72) Erfinder: LEERATANAPHANIT, Sarayut, 63067 Offenbach am Main (DE); BINDER, Matthias, 63571 Gelnhausen (DE); SUCHSLAND, Jens-Peter, 63755 Alzenau (DE)
(74) Vertreter: Hoefer & Partner Patentanwälte mbB
(86) Internationale Anmeldenummer: PCT/EP2020/086474
(87) Internationale Veröffentlichungsnummer: WO 2021/130080

(56) Entgegenhaltungen:
- WO-A1-01/36938
- WO-A1-2007/140714
- WO-A1-2018/156223
- KR-A- 20160 022 027
- US-B2- 7 594 425

## Beschreibung

### Beschreibung

Die Erfindung betrifft ein Verfahren zur Analyse von Defekten in einer funktionalen Schicht einer elektrochemischen Zelle oder einer elektrochemischen Sensorenanwendung.

In der Qualitätskontrolle von Membranen, Katalysatorschichten oder katalysatorbeschichteten Membranen von elektrochemischen Zellen, werden, um z.B. Defekte in den Membranen oder Katalysatorschichten aufzufinden, visuelle Kontrollen durchgeführt. Nachteilig an visuellen Kontrollen ist, dass oftmals nur an der sichtbaren Oberfläche der Schichten Defekte aufgefunden werden können und z.B. Defekte im Inneren der Schichten unerkannt bleiben, zum Beispiel Defekte mit erhöhter Gas-Permeabilität. Beispielsweise offenbart WO 01/36 938 A1 ein Verfahren und eine Vorrichtung zur Analyse einer funktionalen Schicht, wobei die Prüfgaskammer, die zur ersten Oberfläche der funktionalen Schicht geöffnet ist, eine vordefinierte und nicht variabel einstellbare Breite aufweist.

Es ist Aufgabe der Erfindung ein Verfahren zur Analyse einer funktionalen Schicht einer elektrochemischen Zelle oder einer elektrochemischen Sensorenanwendung anzugeben, das zuverlässig Defekte in der gesamten funktionalen Schicht erkennt, schnell und einfach anwendbar ist und wenig technischen Aufwand benötigt. Darüber hinaus ist es auch eine Aufgabe der vorliegenden Erfindung eine Vorrichtung zur Durchführung des Verfahrens bereitzustellen, die sich durch einen einfachen Aufbau und damit durch eine unkomplizierte Handhabung auszeichnet.

Die Lösung dieser Aufgabe erfolgt durch ein Verfahren und eine Vorrichtung, wie sie in den unabhängigen Ansprüchen definiert sind. Die abhängigen Ansprüche haben vorteilhafte Ausführungsformen und Weiterbildungen zum Inhalt.

Demnach wird die Aufgabe durch ein Verfahren zur Analyse einer funktionalen Schicht einer elektrochemischen Zelle oder einer elektrochemischen Sensorenanwendung durch den kennzeichnenden Teil der unabhängigen Ansprüche gelöst.

Das erfindungsgemäße Verfahren befasst sich insbesondere mit der Analyse von Defekten, wie z.B. Löchern, Dichteunterschieden, Perforationen oder Rissen, die in einer funktionalen Schicht einer elektrochemischen Zelle oder einer elektrochemischen Sensorenanwendung auftreten können. Bei der funktionalen Schicht kann es sich beispielsweise um eine Katalysatorschicht, eine Membran, eine katalysatorbeschichtete Membran oder auch um eine katalysatorbeschichtete Membrane im Verbund mit einem Polymerfilm sowie einer Gasdiffusionsschicht handeln, wobei die funktionale Schicht insbesondere in Brennstoffzellenanwendungen, Elektrolysezellanwendungen oder elektrochemischen Sensorenanwendungen eingesetzt wird. Unter einer elektrochemischen Sensorenanwendung wird dabei beispielsweise ein Kohlenmonoxid-Detektor oder ein Wasserstoff-Detektor unter Anwendung einer elektrochemischen Reaktion verstanden.

Die funktionale Schicht hat eine erste Oberfläche, die auch als Unterseite bezeichnet werden kann, und eine zweite Oberfläche, die auch als Oberseite bezeichnet werden kann. Die erste Oberfläche und die zweite Oberfläche sind flächig ausgebildet und liegen in Schichtdickenrichtung einander gegenüber. Die erste und zweite Oberfläche bilden somit die Außenseiten der funktionalen Schicht.

In einem ersten Verfahrensschritt wird eine vordefinierte Menge an Prüfgas an die erste Oberfläche der funktionalen Schicht geleitet. Das Prüfgas tritt durch Diffusion durch die funktionale Schicht und verlässt die funktionale Schicht auf der zweiten Oberfläche der funktionalen Schicht.

Die Menge an durch die funktionale Schicht getretenem Prüfgas wird anschließend quantitativ ermittelt, und zwar durch eine Detektiereinheit, die an der zweiten Oberfläche der funktionalen Schicht, die der ersten Oberfläche der funktionalen Schicht gegenüberliegt, angeordnet ist. Die Detektiereinheit ist im Einzelnen nicht beschränkt und kann jegliche Detektiereinheit umfassen, die eine quantitative Ermittlung des Prüfgases ermöglicht. Vorteilhafterweise wird die Detektiereinheit dabei an der zweiten Oberfläche örtlich so angebracht, dass sie dem Prüfgasstrom, der zur ersten Oberfläche geleitet wird, gegenüber liegt. Somit können Prüfgasverluste, die durch Diffusion entstehen, minimiert werden. Die Detektiereinheit ist jedoch vorzugsweise nicht mit der zweiten Oberfläche dauerhaft verbunden, sondern steht nur mit der zweiten Oberfläche in Kontakt.

Die Diffusion von Prüfgas durch eine funktionale Schicht, also durch den Querschnitt einer funktionalen Schicht einer elektrochemischen Zelle oder einer elektrochemischen Sensorenanwendung, ist charakteristisch für die Homogenität der funktionalen Schicht. Beispielsweise tritt Prüfgas durch ein Loch oder eine Stelle mit reduzierter Dichte oder reduzierter Dicke in der funktionalen Schicht schneller und vollständiger durch als durch eine defektfreie funktionale Schicht. Daher ist die quantitativ ermittelte Menge an Prüfgas, bezogen auf eine vordefinierte Menge an Prüfgas, die auf die erste Oberfläche der funktionalen Schicht geleitet wird, ein sicherer Beleg für das etwaige Vorliegen eines Defekts in der funktionalen Schicht. Ggf. kann auch die Zeit, die für den Durchtritt der vordefinierten Menge an Prüfgas durch die funktionale Schicht verwandt wird, mit in die Analyse einbezogen werden.

Vorteilhafterweise kann die vordefinierte Menge an Prüfgas zunächst durch einen defektfreien Bereich einer funktionalen Schicht geleitet werden und anschließend durch die Detektiereinheit quantitativ ermittelt werden, wieviel und in welcher Zeit Prüfgas durch die funktionale Schicht getreten und an der zweiten Oberfläche der funktionalen Schicht aus dieser ausgetreten ist. Diese Werte können als Kalibrierwerte oder Vergleichswerte herangezogen werden. Tritt in einem Fall weniger Prüfgas (ggf. pro Zeiteinheit) durch die funktionale Schicht als durch den Vergleichswert vorgegeben, spricht dies für eine erhöhte Dichte oder einen gasundurchlässigen Fremdkörper in dem untersuchten Bereich der funktionalen Schicht. Tritt in einem anderen Fall mehr Prüfgas (ggf. pro Zeiteinheit) durch die funktionale Schicht als durch den Vergleichswert vorgegeben, spricht dies für einen Defekt, also z.B. eine Stelle mit reduzierter Dichte, mit reduziertem Querschnitt, mit Loch oder Riss.

Durch das quantitative Ermitteln einer durch eine funktionale Schicht getretenen vordefinierten Menge an Prüfgas, wird ein Verfahren zur Analyse der funktionalen Schicht bereitgestellt, das schnell, einfach und ohne hohen technischen Aufwand anwendbar ist und dabei eine zuverlässige Detektion von etwaigen Defekten in der funktionalen Schicht ermöglicht. Die durch die Detektiereinheit ermittelte Menge an durch die funktionale Schicht getretenem Prüfgas kann beispielsweise über eine Anzeigevorrichtung angezeigt werden.

Gemäß einer vorteilhaften Weiterbildung wird das an die erste Oberfläche der funktionalen Schicht geleitete Prüfgas in einer an der ersten Oberfläche der funktionalen Schicht angeordneten Prüfgaskammer bereitgestellt. Die Prüfgaskammer ist ein abgeschlossener Bereich, der während des Durchführens des Verfahrens lediglich zur ersten Oberfläche der funktionalen Schicht hin geöffnet ist, ggf. mit Ausnahme einer Prüfgaszuführung in die Prüfgaskammer. Die Prüfgaskammer stellt sicher, dass das Prüfgas direkt an die erste Oberfläche der funktionalen Schicht geleitet wird und nicht vor dem Durchtritt durch die funktionale Schicht weg diffundiert. Auch wird somit die Zuführung anderer Gases als das Prüfgas verhindert.

Die Prüfgaskammer hat ein vordefiniertes Volumen sowie eine Öffnung, die in Längsrichtung der funktionalen Schicht eine definierte Länge aufweist. Unter der Längsrichtung der funktionalen Schicht wird dabei eine, gemäß des kartesischen Koordinatensystems, sich in X-Richtung ausdehnende Erstreckung der funktionalen Schicht verstanden, die senkrecht zur Schichtdickenrichtung verläuft. Zudem hat die Öffnung der Prüfgaskammer eine variabel einstellbare Breite, die sich in Querrichtung der funktionalen Schicht erstreckt. Unter der Querrichtung der funktionalen Schicht wird dabei eine, gemäß dem kartesischen Koordinatensystem, sich in Z-Richtung ausdehnende Erstreckung der funktionalen Schicht verstanden, die senkrecht zur Schichtdickenrichtung und auch senkrecht zur Längsrichtung verläuft. Somit kann eine vordefinierte Menge an Prüfgas aus der Prüfgaskammer über die Öffnung der Prüfgaskammer auf die erste Oberfläche der funktionalen Schicht besonders gut einstellbar geleitet werden.

Weiter vorteilhaft wird die funktionale Schicht auf einer Transportvorrichtung bereitgestellt. Als Transportvorrichtung kommt vorteilhafterweise insbesondere ein Förderband in Frage, das die funktionale Schicht horizontal bewegt. Die Transportvorrichtung ist zwischen der Detektiereinheit und der Prüfgaskammer angeordnet, also genauer gesagt zwischen der funktionalen Schicht und der Prüfgaskammer oder zwischen der funktionalen Schicht und der Detektiereinheit, so dass die funktionale Schicht zwischen Prüfgaskammer und Detektiereinheit bewegbar bzw. förderbar ist. Damit das Prüfgas wie vorgesehen durch die funktionale Schicht treten kann, weist die Transportvorrichtung eine Aussparung auf, die dieselben Abmessungen aufweist, wie die zur ersten Oberfläche gerichtete Öffnung der Prüfgaskammer, die durch die Länge in Längsrichtung und die Breite in Querrichtung der Prüfgaskammer definiert wird.

Gemäß einer weiteren vorteilhaften Ausführungsform wird die funktionale Schicht kontinuierlich mittels der Transportvorrichtung zwischen der Prüfgaskammer und der Detektiereinheit, insbesondere mit einer Geschwindigkeit von 0,2 m/min bis 50 m/min, durchgeführt. Das kontinuierliche Transportieren der funktionalen Schicht zwischen die Prüfgaskammer und die Detektiereinheit ermöglicht eine kontinuierliche Analyse der funktionalen Schicht, insbesondere auch dann, wenn die funktionale Schicht in Form einer länglichen Bahn vorliegt. Eine Transportgeschwindigkeit von 0,2 m/min und 50 m/min ermöglicht ferner eine schnelle aber auch zuverlässige Analyse auch über die gesamte Länge der funktionalen Schicht.

Um Fehler bei der Ermittlung der durch die funktionale Schicht getretenen Menge an Prüfgas zu vermeiden, wird die funktionale Schicht vorzugsweise durch Unterdruck an die Transportvorrichtung angesaugt. Somit können Luftspalte zwischen der funktionalen Schicht und der Transportvorrichtung vermieden werden, die eine Wegdiffusion von Prüfgas vor dem Durchtritt des Prüfgases durch die funktionale Schicht begünstigen könnten. Hierzu ist die Transportvorrichtung insbesondere porös, also mit Öffnungen versehen, an die eine Unterdruckvorrichtung anschließbar oder anordenbar ist.

Gemäß einer weiteren Ausführungsform wird die funktionale Schicht über einen feststehenden Walzenkern geführt, auf dem eine bewegliche, poröse Ummantelung angeordnet ist, durch die die funktionale Schicht mittels Unterdruck angesaugt werden kann. Die Prüfgaskammer ist dabei an dem feststehenden Walzenkern angeordnet und zwar insbesondere so, dass die Öffnung der Prüfgaskammer zur Oberfläche des Walzenkerns hin ausgerichtet ist, über die die funktionale Schicht auf der porösen Ummantelung läuft. Somit steht auch in dieser Ausführungsform die Öffnung der Prüfgaskammer in direktem Kontakt mit der ersten Oberfläche der funktionalen Schicht, auf die die vordefinierte Menge an Prüfgas geleitet wird.

Zur Ermöglichung einer gewissen Vorspannung der funktionalen Schicht, wird die funktionale Schicht vorteilhaft über mindestens eine weitere Führungswalze geführt, wodurch Messungenauigkeiten verhindert werden können.

Weiter vorteilhaft kann die funktionale Schicht vor der Durchführung der Analyse von einer ersten Vorratsrolle abgerollt und nach der Durchführung der Analyse auf einer zweiten Vorratsrolle aufgerollt werden, was eine kontinuierliche Verfahrensführung ermöglicht.

Vorzugsweise wird die funktionale Schicht in Längsrichtung seitlich geführt, beispielsweise durch ein Begrenzungsband, so dass die vordefinierte Menge an Prüfgas durch eine vorgesehene Stelle in der funktionalen Schicht treten kann, so dass Messwertverfälschungen reduziert werden.

Um die Verfahrensführung zu beschleunigen, wird das durch die funktionale Schicht getretene Prüfgas vorzugsweise aktiv zu der Detektiereinheit geleitet. Dies kann z.B. mittels eines Inertgasstroms erfolgen, der an der zweiten Oberfläche der funktionalen Schicht vorbei und zu der Detektiereinheit geleitet wird. Auch kann auf der Seite der Detektiereinheit eine Unterdruckvorrichtung vorgesehen sein, die das durch die funktionale Schicht getretene und an der zweiten Oberfläche der funktionalen Schicht vorliegende Prüfgas ansaugt und zur Detektiereinheit leitet.

Um die zur ersten Oberfläche der funktionalen Schicht zu leitende vordefinierte Menge an Prüfgas leichter einstellen zu können, wird das Prüfgas vorzugsweise mit konstantem oder regelbarem Druck an die erste Oberfläche der funktionalen Schicht geleitet.

Zur weiteren Beschleunigung des Verfahrens und zur Sicherstellung, dass das Prüfgas durch die funktionale Schicht tritt, ist vorteilhaft vorgesehen, dass das Prüfgas mit einem Überdruck von mindestens 0,1 bar, insbesondere von mindestens 0,5 bar an die erste Oberfläche der funktionalen Schicht geleitet wird.

Zur Minimierung von Messungenauigkeiten kann ferner vorteilhaft vorgesehen sein, dass das Prüfgas mit einem Volumenstrom von 0,1 L/min bis 100 L/min an die erste Oberfläche der funktionalen Schicht geleitet wird. Hierdurch wird eine signifikante Menge an Prüfgas bereitgestellt, die sehr gut quantitativ ermittelt werden kann.

Ebenfalls erfindungsgemäß wird auch eine Vorrichtung zur Durchführung des vorstehend offenbarten Verfahrens beschrieben. Die Vorrichtung umfasst eine Prüfgaskammer zum Leiten einer vordefinierten Menge an Prüfgas an eine erste Oberfläche einer funktionalen Schicht und eine Detektiereinheit zum quantitativen Ermitteln der Menge an durch die funktionale Schicht getretenem Prüfgas.

Da die erfindungsgemäße Vorrichtung zur Durchführung des vorstehend offenbarten erfindungsgemäßen Verfahrens vorgesehen ist, wird in Bezug auf die Definitionen der vorrichtungsrelevanten Merkmale und technischen Details ergänzend Bezug genommen auf die entsprechenden Ausführungen zum erfindungsgemäßen Verfahren.

Somit ist die Prüfgaskammer an der ersten Oberfläche der funktionalen Schicht angeordnet und die Detektiereinheit ist an der zweiten Oberfläche der funktionalen Schicht, die der ersten Oberfläche der funktionalen Schicht gegenüberliegt, angeordnet. Die funktionale Schicht liegt also zwischen der Prüfgaskammer und der Detektiereinheit. Ferner ist die Prüfgaskammer zur ersten Oberfläche der funktionalen Schicht hin geöffnet und weist hierzu eine Öffnung auf, die in Längsrichtung der funktionalen Schicht eine definierte Länge und in Querrichtung eine variabel einstellbare Breite aufweist. Das Prüfgas kann damit über eine vordefinierbar große Öffnung an die erste Oberfläche der funktionalen Schicht geleitet werden kann.

Die erfindungsgemäße Vorrichtung zeichnet sich durch einen unkomplizierten und platzsparenden Aufbau aus und ermöglicht eine zuverlässige und schnelle Analyse von funktionalen Schichten von elektrochemischen Zellen und elektrochemischen Sensorenanwendungen.

Gemäß einer vorteilhaften Ausführungsform beträgt die Länge der Öffnung von 1 mm bis 500 mm. Die Länge wird hierbei unter Annahme eines kartesischen Koordinatensystems in X-Richtung bestimmt.

Als Prüfgas kommt insbesondere Helium oder ein Gasgemisch mit einer Heliumkonzentration von 1 bis < 100 Vol.-% in Frage, da sich Helium und Gasgemische mit Helium durch sehr gute Diffusionseigenschaften auszeichnen.

Vorteilhafterweise umfasst die Detektiereinheit ein Massenspektrometer, da somit nicht nur eine quantitative Analyse sondern auch eine qualitative Analyse durchgeführt und ein etwaiger Einfluss von Fremdgasen auf die Analyse ausgeschlossen werden kann bzw. ermittelt werden kann.

Vorzugsweise umfasst die Vorrichtung mindestens eine seitliche Führung, insbesondere mindestens ein Führungsband, zum seitlichen Führen der funktionalen Schicht in Längsrichtung zwischen die Prüfgaskammer und die Detektiereinheit. Hierdurch kann ein Verrutschen der zu analysierenden funktionalen Schicht verhindert und eine Lokalisierung des Prüfungsgebiets auf der funktionalen Schicht erleichtert werden. Des Weiteren stellt das Führungsband sicher, dass kein Prüfgas im Sinne eines Bypasses seitlich austreten kann.

Um das Einstellen einer vorbestimmten Menge an die erste Oberfläche zu leitendem Prüfgas zu erleichtern, kann die Vorrichtung weiter vorteilhaft eine Druckregelvorrichtung zum Einstellen und Regeln des Drucks des Prüfgases und/oder eine Dosiervorrichtung zum Einstellen des Volumenstroms des Prüfgases umfassen.

Weiter vorteilhaft im Lichte einer Fehlerminimierung bei der Detektion des durch die funktionale Schicht getretenen Prüfgases, ist eine Ansaugvorrichtung zum aktiven Leiten des durch die funktionale Schicht getretenen Prüfgases zu der Detektiereinheit vorgesehen.

Ferner vorteilhaft kann die Vorrichtung eine Vorrichtung zum Einbringen eines Trägergases für das aktive Leiten des durch die funktionale Schicht getretenen Prüfgases zu der Detektiereinheit umfassen. Diese Ausführungsform ist insbesondere vorteilhaft im Lichte der Ausführungsform, in der die Detektiereinheit ein Massenspektrometer umfasst, da somit der Einfluss des Trägergases separat bestimmt werden kann.

Des Weiteren vorteilhaft kann die Vorrichtung eine Transportvorrichtung zum kontinuierlichen Führen der funktionalen Schicht mittels der Transportvorrichtung zwischen der Prüfgaskammer und der Detektiereinheit umfassen. Hierdurch kann das Verfahren mittels der Vorrichtung kontinuierlich und schnell durchgeführt werden. Zudem kann die funktionale Schicht an beliebigen Stellen einfach und zuverlässig analysiert werden.

In der vorhergehenden Ausführungsform ist es besonders vorteilhaft, wenn zudem eine Unterdruckvorrichtung zum Erzeugen eines Unterdrucks zum Ansaugen der funktionalen Schicht an die Transportvorrichtung vorgesehen ist, da so der Einfluss von Fremdgases auf das Analyseergebnis minimiert und damit die Zuverlässigkeit und Genauigkeit des durch die Vorrichtung ausgeführten Verfahrens verbessert werden.

Die Vorrichtung kann weiter vorteilhaft einen feststehenden Walzenkern umfassen, auf dem eine bewegliche, poröse Ummantelung angeordnet ist, wobei die Prüfgaskammer an dem feststehenden Walzenkern angeordnet ist und die funktionale Schicht auf der porösen Ummantelung geführt ist. Die poröse Ummantelung kann beispielsweise eine separate Schicht sein, die um den Walzenkern bewegbar ist.

Zudem kann mindestens eine weitere Führungswalze vorgesehen sein, die das Transportieren und Bereitstellen der funktionalen Schicht verbessert und ein Spannen der funktionalen Schicht zur Verhinderung von Messfehlern durch ungleichmäßiges Aufliegen der funktionalen Schicht ermöglicht.

Weitere Einzelheiten, Vorteile und Merkmale der vorliegenden Erfindung ergeben sich aus nachfolgender Beschreibung von Ausführungsbeispielen anhand der Zeichnung. Es zeigt:
Fig. 1 eine schematische Seitenansicht einer Vorrichtung gemäß einer ersten Ausführungsform und
Fig. 2 eine schematische Seitenansicht einer Vorrichtung gemäß einer zweiten Ausführungsform.

In den Figuren sind nur die wesentlichen Merkmale der Erfindung dargestellt. Alle übrigen Merkmale sind der Übersichtlichkeit halber weggelassen. Ferner beziffern gleiche Bezugszeichen gleiche Elemente bzw. Bauteile.

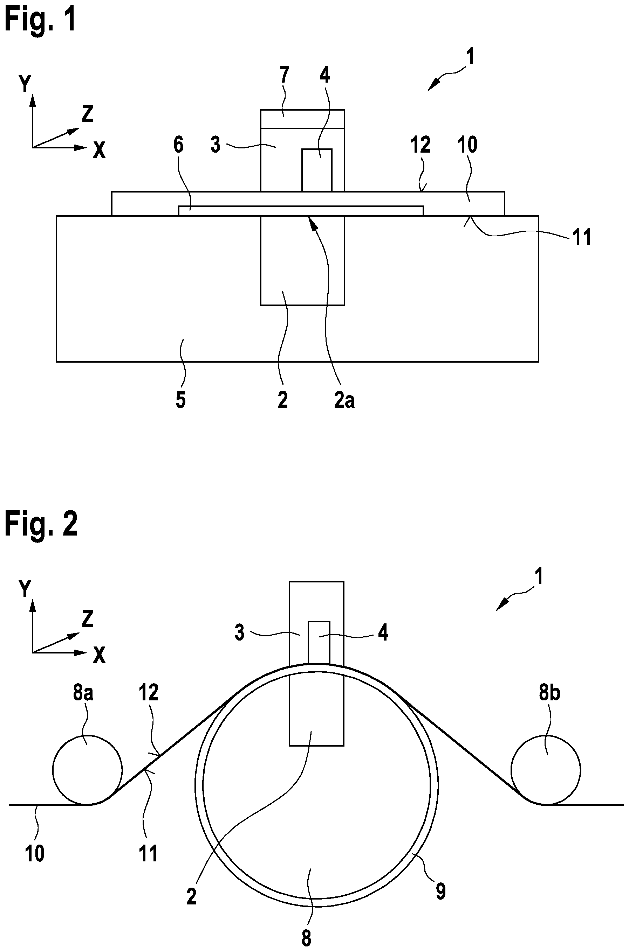

Im Detail zeigt Figur 1 schematisierend eine Vorrichtung 1 zur Durchführung eines Verfahrens zur Analyse einer funktionalen Schicht 10 einer elektrochemischen Zelle oder einer elektrochemischen Sensorenanwendung in Seitenansicht. Die Vorrichtung weist eine Prüfgaskammer 2 zum Leiten einer vordefinierten Menge an Prüfgas an eine erste Oberfläche 11 der funktionalen Schicht 10 und eine Detektiereinheit 3 zum quantitativen Ermitteln der Menge an durch die funktionale Schicht 10 getretenem Prüfgas auf.

Hierbei ist die Prüfgaskammer 2 an der ersten Oberfläche 11 der funktionalen Schicht angeordnet, die in der in Figur 1 gezeigten Ausführungsform die Unterseite der funktionalen Schicht 10 darstellt und sich in XZ-Richtung erstreckt.

Die Detektiereinheit 3 ist an einer zweiten Oberfläche 12 der funktionalen Schicht 10 angeordnet. Die zweite Oberfläche 12 liegt gegenüber der ersten Oberfläche 11 der funktionalen Schicht 10 und bildet in der in Figur 1 dargestellten Ausführungsform die Oberseite der funktionalen Schicht 10, die sich ebenfalls in XZ-Richtung erstreckt.

Die Prüfgaskammer 2 ist zur ersten Oberfläche 11 der funktionalen Schicht 10 hin geöffnet. Sie weist eine Öffnung 2a auf, die in Längsrichtung X der funktionalen Schicht 10, also in der hier gezeigten Ausführungsform in Vorrichtungsdurchlaufrichtung, eine definierte Länge und eine in Z-Richtung verlaufende variabel einstellbare Breite hat. Die Prüfgaskammer 2 steht somit mittels der Öffnung 2a mit der ersten Oberfläche 11 der funktionalen Schicht 10 in Kontakt. Die Öffnung kann z.B. eine Länge von 1 mm bis 500 mm haben.

Die Prüfgaskammer 2 beinhaltet ein Prüfgas, das der Prüfgaskammer 2 z.B. von außen zuführbar ist. Das Prüfgas ist vorzugsweise Helium oder ein Gasgemisch mit einer Heliumkonzentration von 1 bis < 100 Vol.-%.

Zur Ausführung des Verfahrens wird eine vordefinierte Menge an Prüfgas an die erste Oberfläche 11 der funktionalen Schicht 10 geleitet. Hierzu kann das Prüfgas beispielsweise mit einem Druck von mindestens 0,1 bar beaufschlagt werden und die Vorrichtung 1 umfasst hierzu vorteilhafterweise eine Druckregelvorrichtung zum Einstellen und Regeln des Drucks des Prüfgases und/oder eine Dosiervorrichtung zum Einstellen des Volumenstroms des Prüfgases.

Das Prüfgas verlässt die Prüfgaskammer 2 und diffundiert in Schichtdickenrichtung Y durch die funktionale Schicht 10. Zur Beschleunigung der Beförderung des durch die funktionale Schicht 10 getretenen Prüfgases an die Detektiereinheit 3, kann die Vorrichtung 1 eine Ansaugvorrichtung 7 zum aktiven Leiten des durch die funktionale Schicht 10 getretenen Prüfgases zu der Detektiereinheit 3 aufweisen. Das Prüfgas gelangt an die zweite Oberfläche 12 der funktionalen Schicht 10. Dort erfolgt durch die Detektiereinheit 3 das quantitative Ermitteln der Menge an durch die funktionale Schicht 10 getretenem Prüfgas. Die Detektiereinheit 3 kann dazu ein Massenspektrometer 4 umfassen, das die Bestandteile des Prüfgases ermittelt, so dass die Zusammensetzung des Prüfgases und auch Fremdgase bestimmt werden können. Auch kann das durch die Detektiereinheit 3 ermittelte Ergebnis auf einer Anzeigevorrichtung angezeigt werden.

Ist an einer zu analysierenden Stelle der funktionalen Schicht 10 z.B. ein Defekt vorhanden, wie z.B. ein Loch, eine Perforation oder ein Riss, so tritt Prüfgas schneller von der ersten Oberfläche 11 der funktionalen Schicht 10 zur zweiten Oberfläche 12. Mit anderen Worten diffundiert pro Zeiteinheit mehr Prüfgas durch die funktionale Schicht 10 als an defektfreien Stellen der funktionalen Schicht 10. Dies ist quantitativ ermittelbar und kann in Relation zu dem Vorliegen eines Defekts in der funktionalen Schicht gesetzt werden.

Die Vorrichtung 1 zeichnet sich durch eine kompakte Ausführung aus und ermöglicht zuverlässig und schnell eine Analyse einer funktionalen Schicht 10 einer elektrochemischen Zelle oder einer elektrochemischen Sensorenanwendung. Als funktionale Schichten 10 kommen insbesondere Membranen, Katalysatorschichten, katalysatorbeschichtete Membranen und katalysatorbeschichtete Membranen im Verbund mit einem Polymerfilm sowie einer Gasdiffusionsschicht in Frage.

Wie in Figur 1 gezeigt, kann die funktionale Schicht 10 auf einer Transportvorrichtung 5 geführt werden. Die Transportvorrichtung 5 dient dem kontinuierlichen Führen der funktionalen Schicht 10 zwischen der Prüfgaskammer 2 und der Detektiereinheit 3. An der Transportvorrichtung 5 können seitliche Führungen 6 vorgesehen sein, insbesondere in Form eines Führungsbands, so dass die funktionale Schicht 10 seitlich in Längsrichtung X geführt und ein Verrutschen verhindert wird. Zur Verbesserung der Fixierung der funktionalen Schicht 10 an der Transportvorrichtung 5 kann eine Unterdruckvorrichtung zum Erzeugen eines Unterdrucks zum Ansaugen der funktionalen Schicht 10 an die Transportvorrichtung 5 vorgesehen sein.

In dem Bereich der Prüfgaskammer 2 weist die Transportvorrichtung 5 eine entsprechende Ausnehmung auf, so dass das Prüfgas ungehindert aus der Prüfgaskammer 2 an die erste Oberfläche 11 der funktionalen Schicht 10 diffundieren kann.

Die Vorrichtung 1 ist bei hoher Funktionalität kompakt ausgebildet und ermöglicht zuverlässig und schnell eine Analyse der funktionalen Schicht 10 auf Defekte hin.

Figur 2 zeigt eine zweite Ausführungsform der Vorrichtung 1. In der zweiten Ausführungsform umfasst die Vorrichtung 1 anstelle einer Transportvorrichtung 5 einen feststehenden Walzenkern 8, auf dem eine bewegliche, poröse Ummantelung 9 angeordnet ist. Die Prüfgaskammer 2 ist an dem feststehenden Walzenkern 8 angeordnet.

In dieser Ausführungsform wird die funktionale Schicht 10 auf der porösen Ummantelung 9 des feststehenden Walzenkerns 8 geführt. Zusätzlich wird die funktionale Schicht auf zwei weiteren Führungswalzen 8a und 8b geführt, wodurch ein Spannen der funktionalen Schicht 10 ermöglicht wird.

Das Verfahren zur Analyse der funktionalen Schicht 10 auf Defekte kann analog wie in der Vorrichtung 1 aus Figur 1 gezeigt, ausgeführt werden. Über die Führungswalzen 8a und 8b und die poröse Ummantelung 9 wird die funktionale Schicht 10 zwischen die Prüfgaskammer 2 und die Detektiereinheit 3 geleitet. In diesem Bereich wird Prüfgas aus der Prüfgaskammer 2 auf die erste Oberfläche 11 der funktionalen Schicht 10 geleitet, passiert die funktionale Schicht in Schichtdickenrichtung Y und tritt an die zweite Oberfläche 12, in deren unmittelbarer Umgebung die Detektiereinheit 3 angeordnet ist. Das durch die funktionale Schicht 10 getretene Prüfgas wird durch die Detektiereinheit 3 quantitativ ermittelt, so dass auch in dieser Ausführungsform anhand der ermittelten Menge an durch die funktionale Schicht 10 getretenem Prüfgas auf das Vorhandensein von Defekten in der funktionalen Schicht geschlossen werden kann.

Zur Kalibrierung kann in beiden Ausführungsformen durch die Vorrichtung 1 zunächst ein defektfreier Bereich der zu untersuchenden funktionalen Schicht 10 mittels des Verfahrens analysiert werden, so dass ein Vergleichswert für die Menge an durchgetretenem Prüfgas und ggf. auch die dafür benötigte Zeit ermittelt werden kann.

Auch die Vorrichtung der zweiten Ausführungsform zeichnet sich durch eine kompakte aber hoch funktionale Gestaltung aus.

Neben der vorstehenden schriftlichen Beschreibung der Erfindung wird zu deren ergänzender Offenbarung hiermit explizit auf die zeichnerische Darstellung der Erfindung in den Fig. 1 und 2 Bezug genommen.

### Bezugszeichenliste

- 1: Vorrichtung
- 2: Prüfgaskammer
- 2a: Öffnung
- 3: Detektiereinheit
- 4: Massenspektrometer
- 5: Transportvorrichtung
- 6: seitliche Führung
- 7: Ansaugvorrichtung
- 8: feststehender Walzenkern
- 8a: Führungswalze
- 8b: Führungswalze
- 9: poröse Ummantelung
- 10: funktionale Schicht
- 11: erste Oberfläche der funktionalen Schicht
- 12: zweite Oberfläche der funktionalen Schicht
- X: Längsrichtung
- Y: Schichtdickenrichtung
- Z: Querrichtung

## Patentansprüche

1. Verfahren zur Analyse einer funktionalen Schicht (10) einer elektrochemischen Zelle oder einer elektrochemischen Sensorenanwendung, umfassend die Schritte:
- Leiten einer vordefinierten Menge an Prüfgas an eine erste Oberfläche (11) der funktionalen Schicht (10) und
- quantitatives Ermitteln der Menge an durch die funktionale Schicht (10) getretenem Prüfgas durch eine Detektiereinheit (3), die an einer zweiten Oberfläche (12) der funktionalen Schicht (10), die der ersten Oberfläche (11) der funktionalen Schicht (10) gegenüberliegt, angeordnet ist,
wobei das an die erste Oberfläche (11) der funktionalen Schicht (10) geleitete Prüfgas in einer an der ersten Oberfläche (11) der funktionalen Schicht (10) angeordneten Prüfgaskammer (2) bereitgestellt wird, **dadurch gekennzeichnet, dass**
die Prüfgaskammer (2) zur ersten Oberfläche (11) der funktionalen Schicht (10) geöffnet ist und eine Öffnung (2a) aufweist, die in Längsrichtung (X) der funktionalen Schicht (10) eine definierte Länge (2a) und in Querrichtung (Z) eine variabel einstellbare Breite aufweist.

2. Verfahren nach Anspruch 1, wobei die funktionale Schicht (10) auf einer Transportvorrichtung (5) bereitgestellt wird, die zwischen der Detektiereinheit (3) und der Prüfgaskammer (2) angeordnet ist, wobei die Transportvorrichtung (5) eine Aussparung aufweist, die dieselben Abmessungen aufweist wie die Öffnung (2a) der Prüfgaskammer (2).

3. Verfahren nach Anspruch 2, wobei die funktionale Schicht (10) kontinuierlich mittels der Transportvorrichtung (10) zwischen der Prüfgaskammer (2) und der Detektiereinheit (3), insbesondere mit einer Geschwindigkeit von 0,2 m/min bis 50 m/min, durchgeführt wird.

4. Verfahren nach einem der Ansprüche 2 oder 3, wobei die funktionale Schicht (10) an die Transportvorrichtung (5) durch Unterdruck angesaugt wird.

5. Verfahren nach Anspruch 1, wobei die funktionale Schicht (10) über einen feststehenden Walzenkern (8) geführt wird, auf dem eine bewegliche, poröse Ummantelung (9) angeordnet ist, wobei die Prüfgaskammer (2) an dem feststehenden Walzenkern (8) angeordnet ist, wobei die funktionale Schicht (10) insbesondere über mindestens eine weitere Führungswalze (8a, 8b) geführt wird.

6. Verfahren nach einem der vorhergehenden Ansprüche, wobei die funktionale Schicht (10) in Längsrichtung (X) seitlich geführt wird und/oder
wobei das durch die funktionale Schicht (10) getretene Prüfgas aktiv zu der Detektiereinheit (3) geleitet wird und/oder
wobei das Prüfgas mit konstantem oder regelbarem Druck an die erste Oberfläche (11) der funktionalen Schicht (10) geleitet wird und/oder
wobei das Prüfgas mit einem Überdruck von mindestens 0,1 bar, insbesondere von mindestens 0,5 bar an die erste Oberfläche (11) der funktionalen Schicht (10) geleitet wird und/oder
wobei das Prüfgas mit einem Volumenstrom von 0,1 L/min bis 100 L/min an die erste Oberfläche (11) der funktionalen Schicht (10) geleitet wird.

7. Vorrichtung (1) zur Durchführung des Verfahrens nach einem der vorhergehenden Ansprüche umfassend:
- eine Prüfgaskammer (2) zum Leiten einer vordefinierten Menge an Prüfgas an eine erste Oberfläche (11) einer funktionalen Schicht (10) und
- eine Detektiereinheit (3) zum quantitativen Ermitteln der Menge an durch die funktionale Schicht (10) getretenem Prüfgas,
wobei die Prüfgaskammer (2) an der ersten Oberfläche (11) der funktionalen Schicht (10) angeordnet ist und die Detektiereinheit (3) an einer zweiten Oberfläche (12) der funktionalen Schicht (10), die der ersten Oberfläche (11) der funktionalen Schicht (10) gegenüberliegt, angeordnet ist, **dadurch gekennzeichnet, dass**
die Prüfgaskammer (2) zur ersten Oberfläche (11) der funktionalen Schicht (10) hin geöffnet ist und eine Öffnung (2a) aufweist, die in Längsrichtung (X) der funktionalen Schicht (10) eine definierte Länge und in Querrichtung (Z) eine variabel einstellbare Breite aufweist.

8. Vorrichtung nach Anspruch 7, wobei die Öffnung (2a) eine Länge von 1 mm bis 500 mm aufweist und/oder
wobei das Prüfgas Helium oder ein Gasgemisch mit einer Heliumkonzentration von 1 bis < 100 Vol.-% ist und/oder
wobei die Detektiereinheit (3) ein Massenspektrometer (4) umfasst und/oder
ferner umfassend mindestens eine seitliche Führung (6), insbesondere mindestens ein Führungsband, zum seitlichen Führen der funktionalen Schicht (10) in Längsrichtung (X) und/oder
ferner umfassend eine Druckregelvorrichtung zum Einstellen und Regeln des Drucks des Prüfgases und/oder eine Dosiervorrichtung zum Einstellen des Volumenstroms des Prüfgases und/oder
ferner umfassend eine Ansaugvorrichtung (7) zum aktiven Leiten des durch die funktionale Schicht (10) getretenen Prüfgases zu der Detektiereinheit (3) und/oder
ferner umfassend eine Vorrichtung zum Einbringen eines Trägergases für das aktive Leiten des durch die funktionale Schicht (10) getretenen Prüfgases zu der Detektiereinheit (3).

9. Vorrichtung nach Anspruch 7 oder 8, ferner umfassend eine Transportvorrichtung (5) zum kontinuierlichen Führen der funktionalen Schicht (10) mittels der Transportvorrichtung (5) zwischen der Prüfgaskammer (2) und der Detektiereinheit (3), wobei die Vorrichtung insbesondere eine Unterdruckvorrichtung zum Erzeugen eines Unterdrucks zum Ansaugen der funktionalen Schicht (10) an die Transportvorrichtung (5) umfasst.

10. Vorrichtung nach Anspruch 7 oder 8, ferner umfassend einen feststehenden Walzenkern (8), auf dem eine bewegliche, poröse Ummantelung (9) angeordnet ist, wobei die Prüfgaskammer (2) an dem feststehenden Walzenkern (8) angeordnet ist und die funktionale Schicht (10) auf der porösen Ummantelung (9) geführt ist, wobei die Vorrichtung ferner insbesondere ferner mindestens eine weitere Führungswalze (8a, 8b) umfasst.

## Claims

1. A method for analyzing a functional layer (10) of an electrochemical cell or an electrochemical sensor application, comprising the steps of:
- passing a predefined amount of test gas to a first surface (11) of the functional layer (10), and
- quantitatively determining the amount of test gas that has passed through the functional layer (10) by a detection unit (3) that is arranged on a second surface (12) of the functional layer (10) that is opposite the first surface (11) of the functional layer (10),
wherein the test gas passed to the first surface (11) of the functional layer (10) is provided in a test gas chamber (2) arranged on the first surface (11) of the functional layer (10), **characterized in that**
the test gas chamber (2) is open to the first surface (11) of the functional layer (10) and has an opening (2a) that has a defined length (2a) in the longitudinal direction (X) of the functional layer (10) and a variably adjustable width in the transverse direction (Z).

2. The method according to claim 1, wherein the functional layer (10) is provided on a transport device (5) that is arranged between the detection unit (3) and the test gas chamber (2), wherein the transport device (5) has a recess that has the same dimensions as the opening (2a) of the test gas chamber (2).

3. The method according to claim 2, wherein the functional layer (10) is continuously carried out by means of the transport device (10) between the test gas chamber (2) and the detection unit (3), in particular at a speed of 0.2 m/min to 50 m/min.

4. The method according to one of claims 2 or 3, wherein the functional layer (10) is sucked onto the transport device (5) by negative pressure.

5. The method according to claim 1, wherein the functional layer (10) is guided over a stationary roll core (8) on which a movable, porous casing (9) is arranged, wherein the test gas chamber (2) is arranged on the stationary roll core (8), wherein the functional layer (10) is guided in particular over at least one further guide roll (8a, 8b).

6. The method according to one of the preceding claims, wherein the functional layer (10) is guided laterally in the longitudinal direction (X) and/or
wherein the test gas that has passed through the functional layer (10) is actively passed to the detection unit (3) and/or
wherein the test gas is passed at constant or controllable pressure to the first surface (11) of the functional layer (10) and/or
wherein the test gas is passed at an overpressure of at least 0.1 bar, in particular of at least 0.5 bar, to the first surface (11) of the functional layer (10) and/or
wherein the test gas is passed at a volume flow of 0.1 L/min to 100 L/min to the first surface (11) of the functional layer (10).

7. A device (1) for carrying out the method according to one of the preceding claims, comprising:
- a test gas chamber (2) for passing a predefined amount of test gas to a first surface (11) of a functional layer (10), and
- a detection unit (3) for quantitatively determining the amount of test gas that has passed through the functional layer (10),
wherein the test gas chamber (2) is arranged on the first surface (11) of the functional layer (10) and the detection unit (3) is arranged on a second surface (12) of the functional layer (10) that is opposite the first surface (11) of the functional layer (10), **characterized in that**
the test gas chamber (2) is open to the first surface (11) of the functional layer (10) and has an opening (2a) that has a defined length in the longitudinal direction (X) of the functional layer (10) and a variably adjustable width in the transverse direction (Z).

8. The device according to claim 7, wherein the opening (2a) has a length of 1 mm to 500 mm and/or
wherein the test gas is helium or a gas mixture with a helium concentration of 1 to < 100 vol.-% and/or
wherein the detection unit (3) comprises a mass spectrometer (4) and/or
further comprising at least one lateral guide (6), in particular at least one guide belt, for laterally guiding the functional layer (10) in the longitudinal direction (X) and/or
further comprising a pressure control device for setting and controlling the pressure of the test gas and/or a dosing device for setting the volume flow of the test gas and/or
further comprising a suction device (7) for actively passing the test gas that has passed through the functional layer (10) to the detection unit (3) and/or
further comprising a device for introducing a carrier gas for actively passing the test gas that has passed through the functional layer (10) to the detection unit (3).

9. The device according to claim 7 or 8, further comprising a transport device (5) for continuously guiding the functional layer (10) by means of the transport device (5) between the test gas chamber (2) and the detection unit (3), wherein the device comprises in particular a negative pressure device for generating a negative pressure for sucking the functional layer (10) onto the transport device (5).

10. The device according to claim 7 or 8, further comprising a stationary roll core (8) on which a movable, porous casing (9) is arranged, wherein the test gas chamber (2) is arranged on the stationary roll core (8) and the functional layer (10) is guided on the porous casing (9), wherein the device further comprises in particular at least one further guide roll (8a, 8b).

## Revendications

1. Procédé d'analyse d'une couche fonctionnelle (10) d'une cellule électrochimique ou d'une application de capteur électrochimique, comprenant les étapes consistant à :
- diriger une quantité prédéfinie de gaz d'essai vers une première surface (11) de la couche fonctionnelle (10), et
- déterminer quantitativement la quantité de gaz d'essai ayant traversé la couche fonctionnelle (10) au moyen d'une unité de détection (3) qui est disposée sur une deuxième surface (12) de la couche fonctionnelle (10) qui est opposée à la première surface (11) de la couche fonctionnelle (10),
dans lequel le gaz d'essai dirigé vers la première surface (11) de la couche fonctionnelle (10) est fourni dans une chambre de gaz d'essai (2) disposée sur la première surface (11) de la couche fonctionnelle (10), **caractérisé en ce que**
la chambre de gaz d'essai (2) est ouverte vers la première surface (11) de la couche fonctionnelle (10) et présente une ouverture (2a) qui présente une longueur définie (2a) dans la direction longitudinale (X) de la couche fonctionnelle (10) et une largeur réglable de manière variable dans la direction transversale (Z).

2. Procédé selon la revendication 1, dans lequel la couche fonctionnelle (10) est fournie sur un dispositif de transport (5) qui est disposé entre l'unité de détection (3) et la chambre de gaz d'essai (2), dans lequel le dispositif de transport (5) présente un évidement qui présente les mêmes dimensions que l'ouverture (2a) de la chambre de gaz d'essai (2).

3. Procédé selon la revendication 2, dans lequel la couche fonctionnelle (10) est réalisée en continu au moyen du dispositif de transport (10) entre la chambre de gaz d'essai (2) et l'unité de détection (3), en particulier à une vitesse de 0,2 m/min à 50 m/min.

4. Procédé selon l'une quelconque des revendications 2 ou 3, dans lequel la couche fonctionnelle (10) est aspirée sur le dispositif de transport (5) par dépression.

5. Procédé selon la revendication 1, dans lequel la couche fonctionnelle (10) est guidée par le biais d'un noyau de rouleau fixe (8) sur lequel est disposée une enveloppe poreuse mobile (9), dans lequel la chambre de gaz d'essai (2) est disposée sur le noyau de rouleau fixe (8), dans lequel la couche fonctionnelle (10) est guidée en particulier par le biais d'au moins un rouleau de guidage supplémentaire (8a, 8b).

6. Procédé selon l'une quelconque des revendications précédentes, dans lequel la couche fonctionnelle (10) est guidée latéralement dans la direction longitudinale (X) et/ou
dans lequel le gaz d'essai ayant traversé la couche fonctionnelle (10) est dirigé activement vers l'unité de détection (3) et/ou
dans lequel le gaz d'essai est dirigé avec une pression constante ou réglable vers la première surface (11) de la couche fonctionnelle (10) et/ou
dans lequel le gaz d'essai est dirigé avec une surpression d'au moins 0,1 bar, en particulier d'au moins 0,5 bar, vers la première surface (11) de la couche fonctionnelle (10) et/ou
dans lequel le gaz d'essai est dirigé avec un débit volumique de 0,1 L/min à 100 L/min vers la première surface (11) de la couche fonctionnelle (10).

7. Dispositif (1) pour la mise en oeuvre du procédé selon l'une quelconque des revendications précédentes, comprenant :
- une chambre de gaz d'essai (2) pour diriger une quantité prédéfinie de gaz d'essai vers une première surface (11) d'une couche fonctionnelle (10), et
- une unité de détection (3) pour déterminer quantitativement la quantité de gaz d'essai ayant traversé la couche fonctionnelle (10),
dans lequel la chambre de gaz d'essai (2) est disposée sur la première surface (11) de la couche fonctionnelle (10) et l'unité de détection (3) est disposée sur une deuxième surface (12) de la couche fonctionnelle (10) qui est opposée à la première surface (11) de la couche fonctionnelle (10), en ce que
la chambre de gaz d'essai (2) est ouverte vers la première surface (11) de la couche fonctionnelle (10) et présente une ouverture (2a) qui présente une longueur définie dans la direction longitudinale (X) de la couche fonctionnelle (10) et une largeur réglable de manière variable dans la direction transversale (Z).

8. Dispositif selon la revendication 7, dans lequel l'ouverture (2a) présente une longueur de 1 mm à 500 mm et/ou
dans lequel le gaz d'essai est de l'hélium ou un mélange de gaz avec une concentration en hélium de 1 à < 100 % en volume et/ou
dans lequel l'unité de détection (3) comprend un spectromètre de masse (4) et/ou
comprenant en outre au moins un guidage latéral (6), en particulier au moins une bande de guidage, pour le guidage latéral de la couche fonctionnelle (10) dans la direction longitudinale (X) et/ou
comprenant en outre un dispositif de régulation de pression pour le réglage et la régulation de la pression du gaz d'essai et/ou un dispositif de dosage pour le réglage du débit volumique du gaz d'essai et/ou
comprenant en outre un dispositif d'aspiration (7) pour diriger activement le gaz d'essai ayant traversé la couche fonctionnelle (10) vers l'unité de détection (3) et/ou
comprenant en outre un dispositif pour l'introduction d'un gaz porteur pour diriger activement le gaz d'essai ayant traversé la couche fonctionnelle (10) vers l'unité de détection (3).

9. Dispositif selon la revendication 7 ou 8, comprenant en outre un dispositif de transport (5) pour le guidage en continu de la couche fonctionnelle (10) au moyen du dispositif de transport (5) entre la chambre de gaz d'essai (2) et l'unité de détection (3), dans lequel le dispositif comprend en particulier un dispositif de dépression pour générer une dépression pour aspirer la couche fonctionnelle (10) sur le dispositif de transport (5).

10. Dispositif selon la revendication 7 ou 8, comprenant en outre un noyau de rouleau fixe (8) sur lequel est disposée une enveloppe poreuse mobile (9), dans lequel la chambre de gaz d'essai (2) est disposée sur le noyau de rouleau fixe (8) et la couche fonctionnelle (10) est guidée sur l'enveloppe poreuse (9), dans lequel le dispositif comprend en outre en particulier au moins un rouleau de guidage supplémentaire (8a, 8b).
